# FASCICULE DE BREVET EUROPEEN

(11) **EP 0 856 293 B1**
(45) Date de publication et mention de la délivrance du brevet: **28.05.2003**
(21) Numéro de dépôt: 98400220.4
(22) Date de dépôt: 03.02.1998
(51) Int. Cl.: A61B 17/86

(54) **Dispositif de vis à tête filetée destiné à pernettre la fixation de petits fragments d'os**
Knochenschraube zum Verbinden von Knochenteilchen
Bone screw for connecting small bone fragments

(30) Priorité: 04.02.1997 FR 9701386
(43) Date de publication de la demande: 05.08.1998
(73) Titulaire: S.C.I. DIGO, 2550 Luxembourg (LU)
(72) Inventeur: Diebold, Patrice Francois, 54000 Nancy (FR)
(74) Mandataire: Martin, Didier

(56) Documents cités:
- EP-A- 0 657 142
- DE-A- 19 529 901
- FR-A- 2 722 086
- US-A- 4 463 753
- US-A- 4 858 601

## Description

La présente invention a pour objet un dispositif de vis à tête filetée destiné à permettre la fixation de petits fragments d'os.

La fixation de petits fragments d'os, lors de la rupture de la solution de continuité osseuse, pose des problèmes aux praticiens étant donnée la dimension des os concernés, ladite rupture pouvant être soit traumatisante dans le cas d'une fracture, soit provoquée dans le cas d'une intervention chirurgicale.

Pour assurer une ostéogénèse rapide, afin de former un cal osseux dont la qualité mécanique permet le retour à une fonction normale, la fixation des deux fragments d'os doit être réalisée conjointement avec une compression.

Dans le traitement curatif chirurgical de l'hallux valgus, il est fréquent de réaliser une ostéotomie qu'elle soit en chevron ou dite de Scarf, suivie d'une fixation osseuse.

Dans la pratique, le chirurgien procède à une coupe osseuse visant à redonner un axe physiologique au métatarsien en décalant les deux fragments d'os latéralement et/ou longitudinalement, puis il fixe lesdits fragments dans cette position.

On notera qu'il en est de même pour la chirurgie de la main, notamment dans le traitement des fractures ou pseudarthrose du scaphoïde. En effet, ce petit os situé à la base du pouce a un rôle important dans la fonction même de celui-ci. Sa consolidation est difficile et dans tous les cas inexistante si les fragments ne sont pas stables.

Pour fixer deux fragments d'os et obtenir une consolidation osseuse, le chirurgien a le choix entre plusieurs modes de fixation.

La fixation par broche est aisée, mais la stabilité n'est pas assurée d'une façon certaine, et les contraintes de mise en charge peuvent nuire à la fixation, la non consolidation des fragments osseux entraînant pour le patient une impotence telle qu'il doit être réopéré.

On peut aussi employer des agrafes, mais la topographie anatomique et la qualité osseuse diminuent la facilité de mise en place, et il est de plus impossible d'obtenir une fixation en compressions.

Dans tous les cas, aucune partie de l'implant mis en place ne doit faire saillie de la matière osseuse, les tissus environnants seraient alors agressés lors des mouvements, le patient ressentirait sa présence et en souffrirait.

Pour pallier ces inconvénients, il a été proposé d'utiliser une vis du type de celles utilisées pour la coaptation de gros os et qui sont décrites par exemple dans le brevet U. S. 4 175 555 et le document WO-A-91 09572. Une telle vis comprend trois parties, une partie distale filetée, une partie intermédiaire lisse et une partie proximale, ou tête, filetée, le filetage de ladite partie proximale étant d'un pas plus petit que celui du filetage de ladite partie distale.

Le vissage d'une telle vis, dans un trou préalablement foré et taraudé dans les deux fragments d'os à solidariser, a pour effet, du fait de la différence des pas, une pénétration plus rapide de la partie distale, ce qui entraîne un resserrement des deux fragments d'os.

On connaît également, par le document FR-A-2 722 086, une vis de ce type plus particulièrement destinée à la coaptation de petits fragments d'os pour le traitement chirurgical de l'hallux valgus.

Le préambule de la revendication 1 est basé sur ce document.

Cette vis présente toutefois des inconvénients notamment en ce qui concerne sa mise en place et la compressions obtenue.

La mise en place d'une telle vis nécessite l'emploi d'une mèche étagée et canulée, c'est-à-dire une mèche comportant d'une part deux parties de diamètres différents pour permettre le forage de l'emplacement de la partie distale et celui de la partie proximale de la vis, et d'autre part percée axialement d'un canal permettant, lors du forage , le guidage de ladite mèche sur une broche qui maintient les fragments d'os dans la position requise.

Or une telle mèche, du fait de ses faibles dimensions, est très fragile, et il est fréquent qu'elle casse au niveau du point de jonction de ses deux parties de diamètres différents, ce qui entraîne des complications lors de l'intervention chirurgicale.

D'autre part, la partie intermédiaire lissée de la vis est pratiquement inexistante, en tous cas insuffisante pour permettre le coulissement des fragments d'os afin de réaliser une bonne compression.

Par ailleurs, la différence de pas entre le filetage de la partie proximale et la partie distale n'est pas assez importante pour permettre une bonne compression des deux fragments d'os. Cet inconvénient est d'ailleurs lié au fait que la partie lisse est courte, en effet une différence de pas importante, permettant une grande compression, est incompatible avec une faible possibilité de coulissement.

Le brevet U. S. 4 858 601 décrit un dispositif à vis comportant une partie distale filetée, une partie proximale filetée et une partie médiane non filetée. Pour effectuer la compression des os à solidariser, les parties proximale et distale sont tournées indépendamment l'une de l'autre. On introduit la vis dans les os, on fait tourner la partie distale dans un sens à l'aide d'un outil, puis la partie proximale dans le sens opposé à l'aide d'un autre outil de manière à amener les os en compression.

Un tel dispositif a pour inconvénient de nécessiter deux outils pour faire tourner la vis et en outre ce dispositif ne comporte pas de moyen de taraudage ni de forage.

Le brevet U.S. 4 640 271 décrit une vis qui comporte à son extrémité distale une première série de filetage et à son extrémité proximale une deuxième série de filetage de pas supérieur au filetage de l'extrémité distale. Le deuxième filetage est placé sur la partie intermédiaire lisse de la vis et peut coulisser librement sur celle-ci. Une telle vis est utilisée pour consolider des cols du fémur.

Le brevet U. S. 5 019 079 décrit une vis comportant une extrémité distale et une extrémité proximale séparée par une portion lisse intermédiaire, le pas de l'extrémité distale étant plus grand que celui de l'extrémité proximale. Le diamètre externe de l'extrémité distale est sensiblement égal au diamètre de la portion lisse et le diamètre de l'extrémité proximale est supérieur au diamètre de la portion lisse. Une telle vis n'est pas autotaraudeuse ni autoforeuse.

Le document DE-A-195 29 901 décrit une vis du type vis canulée à trois parties. Cette vis n'est ni autotaraudeuse ni autoforeuse.

Tous ces dispositifs nécessitent d'effectuer un trou préalablement foré et taraudé dans les deux fragments d'os à solidariser. Or, ceci demande une grande précision de la part du praticien qui doit réaliser à l'aide d'un outil ce trou foré et taraudé.

La présente invention a pour but de remédier à ces divers inconvénients en proposant un dispositif de vis permettant une fixation avec une compression importante, et dont la pose est aisée et rapide, ce qui diminue le temps opératoire.

Les objets assignés à l'invention sont atteints à l'aide d'un dispositif de vis tel que celui revendiqué dans la revendication 1.

Le dispositif de vis selon l'invention, qui est du type vis canulée à trois parties, à savoir une partie proximale filetée, une partie intermédiaire lisse et une partie distale filetée, ladite partie proximale étant d'un diamètre supérieur à celui de ladite partie distale, et son filetage étant d'un pas inférieur à celui de ladite partie distale, se caractérise essentiellement en ce que d'une part ladite partie distale comporte deux filetages chacun d'un pas au moins double de celui du filetage de ladite partie proximale; d'autre part la partie intermédiaire lisse est de préférence d'une longueur d'environ un tiers de la longueur totale de la vis; d'autre part encore les extrémités distales desdites parties proximale et distale sont de préférence autoforeuses et autotaraudeuses, c'est-à-dire qu'elles sont chanfreinées à environ 90 degrés et comportent chacune trois entailles longitudinales pratiquées profondément jusque dans les racines desdites parties.

Un dispositif de vis selon l'invention permet ainsi de réaliser une coaptation de petits fragments d'os avec une bonne compression, sans avoir recourt à un forage préalable au moyen d'une mèche étagée.

Le double filetage de la partie distale permet d'obtenir une bonne fixation dans l'os spongieux en compensant la taille du pas.

Selon un autre mode de réalisation de la présente invention le dispositif est tel que la partie distale est chanfreinée d'un angle double de l'angle selon lequel la partie proximale est chanfreinée.

Selon un mode de réalisation plus préféré de la présente invention, le dispositif selon l'invention est tel que la partie proximale est chanfreinée à 60 degrés et la partie distale est chanfreinée à 120 °.

Selon encore un autre mode de réalisation de la présente invention, la partie distale peut être biseautée d'un angle compris entre 20 et 50° et la partie distale ne comporte pas d'entaille longitudinale.

Les avantages et les caractéristiques de la présente invention ressortiront plus clairement de la description qui suit, qui se rapporte au dessin annexé, lequel représente un mode de réalisation non limitatif.

Dans les dessins annexés
La Figure 1 représente une vue en perspective d'un dispositif de vis selon l'invention.
La Figure 2 représente une vue en perspective sous un autre angle et à plus grande échelle du dispositif de la Figure 1.
La Figure 3 représente une vue en plan avec coupes partielles du dispositif de la Figure 1.
La Figure 4 représente une vue en coupe selon l'axe XX de la Figure 3.
La Figure 5 représente une vue en plan de l'extrémité distale du dispositif de la Figure 1.

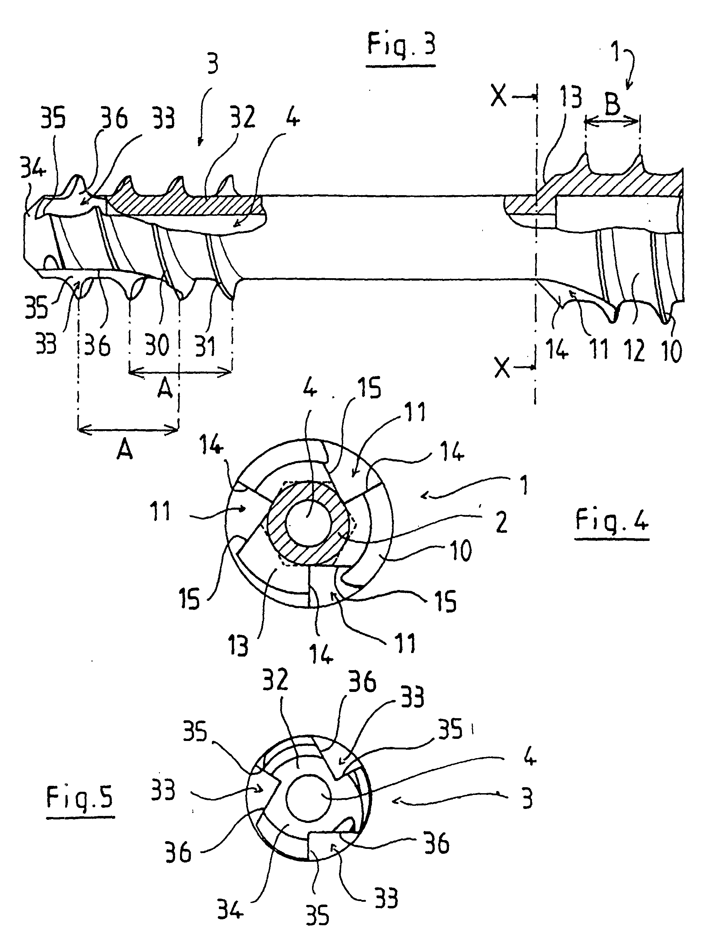

La Figure 3a représente une vue en plan avec coupes partielles d'un autre mode de réalisation du dispositif de l'invention.
La Figure 4a représente une vue en coupe selon l'axe AA de la Figure 3a.
La Figure 5a représente une vue en plan de l'extrémité proximale du dispositif de la Figure 3a.

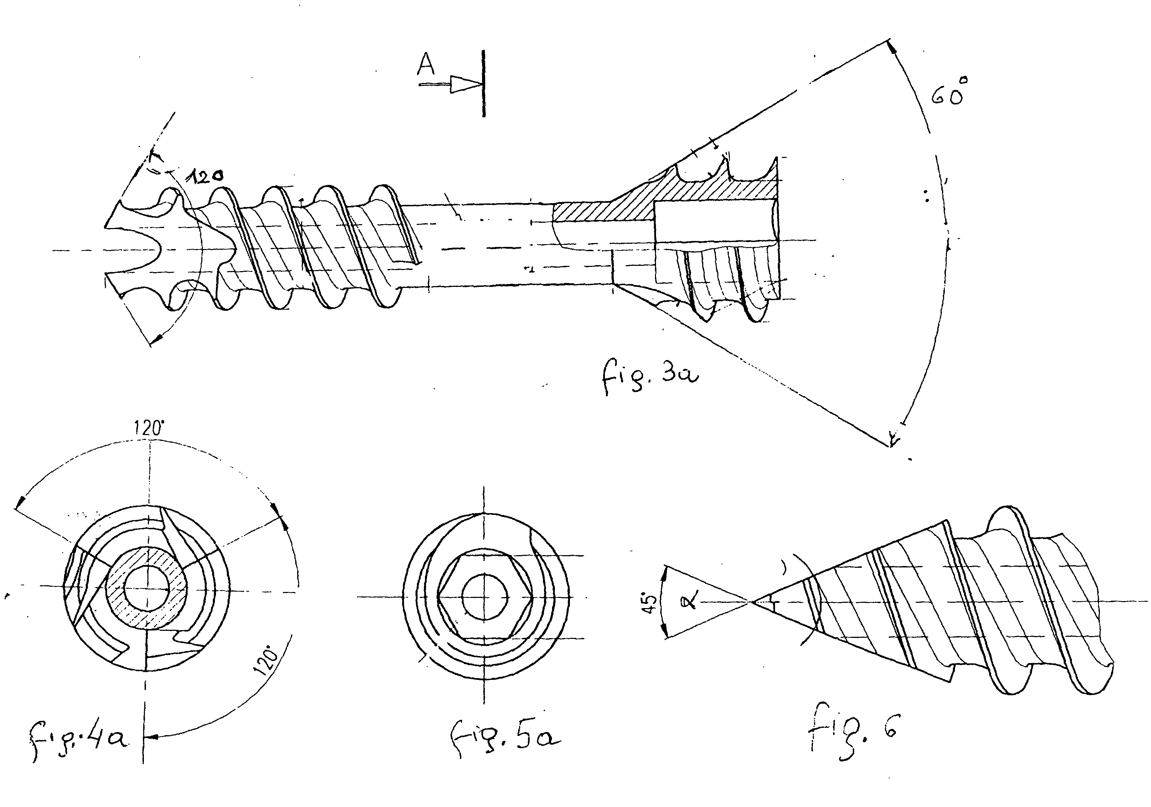

La Figure 6 représente une vue de l'extrémité distale d'un autre mode de réalisation du dispositif selon la présente invention.

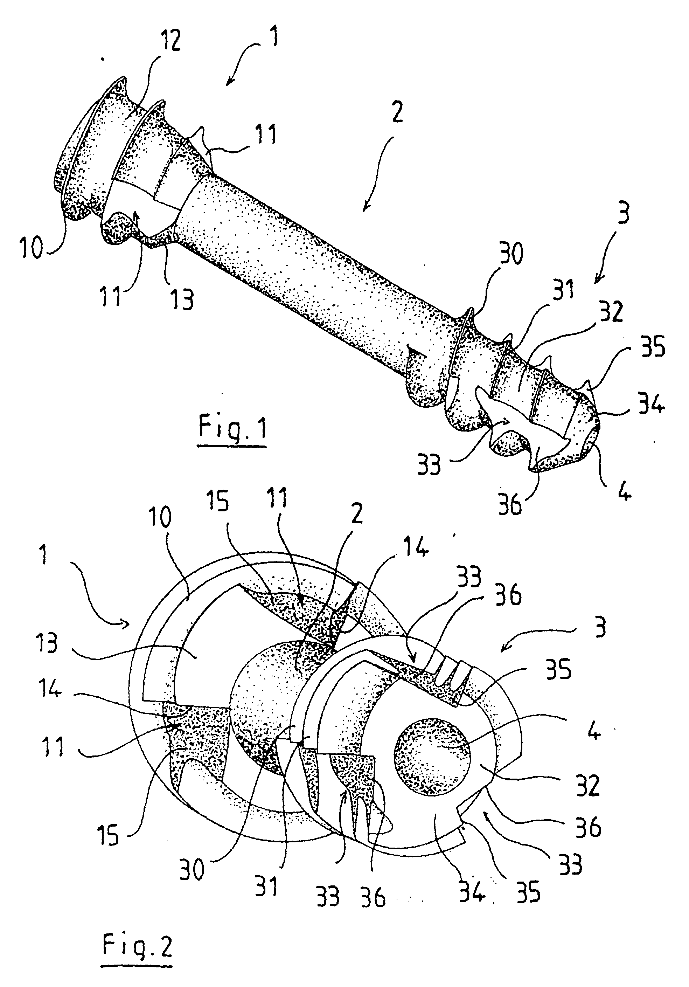

Si on se réfère aux Figures 1, 2 et 3, on peut voir qu'un dispositif de vis selon l'invention comporte trois parties, une partie proximale 1, une partie intermédiaire 2 et une partie distale 3 et qu'elle est traversée axialement par un canal 4.

La partie proximale 1 comporte un filetage 10, tandis que la partie intermédiaire 2 est lisse et que la partie distale 3 comporte deux filetages 30 et 31.

La partie intermédiaire lisse 2 est d'une longueur correspondant à environ un tiers de la longueur totale de la vis, et son diamètre est égal à celui de la racine 32 de la partie distale 3.

Comme on peut le voir sur la Figure 3, le pas A des filetages 30 et 31 est double du pas B du filetage 10, ce qui permet une grande compression lors du vissage.

A titre d'exemple, une vis selon l'invention d'une longueur de 10 mm, dont la partie proximale 1 est d'une longueur de 3,5 mm avec un filetage 10 d'un pas de 1,25 mm permet une compression d'environ 2 mm alors qu'une vis telle que celle décrite dans le document FR-A-2 722 086 de même longueur n'autorise qu'une compression de 0,8 mm.

Si on se réfère également à la Figure 4, on peut voir que la partie proximale 1 comporte trois entailles 11 pratiquées longitudinalement dans son filetage 10 et sa racine 12, et son extrémité distale comporte un chanfrein 13 à 90 degrés, ce qui permet à la fois le forage et le taraudage.

De même on peut voir sur la Figure 5 que la partie distale 3 comporte trois entailles 33, pratiquées longitudinalement dans les filetages 30 et 31 et profondément dans la racine 32, jusqu'à proximité du canal 4, et son extrémité comporte un chanfrein 34 à 90 degrés.

Les entailles 11, respectivement 33, comportent chacune deux bords 14 et 15, respectivement 35 et 36, faisant entre eux un angle droit, le bord d'attaque 14, respectivement 35, étant radial à l'axe de la vis.

Le diamètre de la racine 12 de la partie proximale 1 est préférentiellement égal à celui hors tout de la partie distale 3, ce qui permet de limiter la profondeur des entailles 11 dans la racine 12 à la différence d'épaisseur entre celle-ci et la racine 32, en sorte que la jonction entre la partie intermédiaire 2 et la partie proximale 1 n'est pas fragilisée.

Cela permet d'autre part un forage aisé de la corticale par la partie proximale 1, puisqu'il consiste uniquement à forer une partie taraudée.

On notera que la partie proximale 1 comporte une empreinte creuse représentée en traits discontinus sur la Figure 4, permettant le vissage de la vis.

Selon un autre mode de réalisation de la présente invention, l'extrémité proximale est chanfreinée à 60 degrés et l'extrémité distale est chanfreinée à 120 degrés, comme on peut le voir sur les Figures 3a, 4a et 5a.

Selon encore un autre mode de réalisation selon l'invention, la partie distale, comme on peut le voir sur la figure 6, est biseautée d'un angle a compris entre 20 et 50 degrés, de préférence de 45° comme représenté sur la figure 6. Par ailleurs, dans ce cas, la partie distale ne comporte pas d'entaille longitudinale.

## Revendications

1. Dispositif de vis à tête filetée destiné à permettre la fixation de petits fragments d'os, comprenant une partie proximale filetée (1), une partie intermédiaire lisse (2) et une partie distale filetée (3), ladite partie proximale (1) étant d'un diamètre supérieur à celui de ladite partie distale (3), et son filetage (10) étant d'un pas inférieur à celui de ladite partie distale (3), **caractérisé en ce que** ladite partie distale (3) comporte deux filetages (30, 31) chacun d'un pas (A) au moins double de celui (B) du filetage (10) de ladite partie proximale (1).

2. Dispositif selon la revendication 1, **caractérisé en ce que** ladite partie intermédiaire lisse (2) est d'une longueur d'environ un tiers de la longueur totale de la vis.

3. Dispositif selon la revendication 1 ou 2, **caractérisé en ce que** les extrémités distales desdites parties proximale et distale sont autoforeuses et autotaraudeuses, c'est-à-dire qu'elles sont chanfreinées et comportent chacune trois entailles longitudinales (11, 33) pratiquées profondément jusque dans les racines (12, 13) desdites parties (1, 3).

4. Dispositif selon l'une des revendications 1 à 3, **caractérisé en ce que** ladite partie distale est chanfreinée d'un angle égal au double de celui de ladite partie proximale.

5. Dispositif selon l'une des revendications 1 à 4, **caractérisé en ce que** ladite partie proximale est chanfreinée à environ 60 degrés et ladite partie distale est chanfreinée à environ 120 degrés. .

6. Dispositif selon l'une des revendications 1 à 3, **caractérisé en ce que** la partie proximale et la partie distale sont chanfreinées chacune à environ 90 degrés.

7. Dispositif selon l'une des revendications 1 à 2, **caractérisé par le fait que** ladite partie distale est biseautée d'un angle compris entre 20 et 50° et qu'elle ne comporte pas d'entaille.

8. Dispositif selon l'une des revendications 1 à 6, **caractérisé en ce que** le diamètre de la racine (12) de ladite partie proximale (1) est égal à celui hors tout de la partie distale (3).

## Claims

1. Threaded-head screw device intended to allow the fixing of small fragments of bone, comprising a threaded proximal part (1), a smooth intermediate part (2) and a threaded distal part (3), the said proximal part (1) having a diameter greater than that of the said distal part (3), and its thread (10) having a pitch less than that of the said distal part (3), **characterised in that** the said distal part (3) has two threads (30, 31), each with a pitch (A) at least twice that (B) of the thread (10) on the said proximal part (1).

2. Device according to Claim 1, **characterised in that** the said smooth intermediate part (2) has a length approximately one third of the total length of the screw.

3. Device according to Claim 1 or 2, **characterised in that** the distal ends of the said proximal and distal parts are self-drilling and self-tapping, that is to say they are bevelled and each have three longitudinal cuts (11, 33) made deep into the roots (12, 13) of the said parts (1, 3).

4. Device according to any of Claims 1 to 3, **characterised in that** the said distal part is bevelled by an angle equal to twice that of the said proximal part.

5. Device according to any of Claims 1 to 4, **characterised in that** the said proximal part is bevelled at approximately 60 degrees and the said distal part is bevelled at approximately 120 degrees.

6. Device according to any of Claims 1 to 3, **characterised in that** the proximal part and the distal part are each bevelled at approximately 90 degrees.

7. Device according to any of Claims 1 to 2, **characterised by** the fact that the said distal part is bevelled by an angle of between 20 and 50° and in that it does not have any cut.

8. Device according to any of Claims 1 to 6, **characterised in that** the diameter of the root (12) of the said proximal part (1) is equal to the overall diameter of the distal part (3).

## Patentansprüche

1. Knochenschraube zum Verbinden von Knochenteilchen mit einem vorderen Abschnitt (1) mit Gewinde, einem mittleren glatten Abschnitt (2) und einem hinteren Abschnitt (3) mit Gewinde, wobei der vordere Abschnitt (1) einen größeren Durchmesser als der hintere Abschnitt (3) aufweist und sein Gewinde einen Gewindering weniger als der hintere Abschnitt (3) aufweist, **dadurch gekennzeichnet, daß** der hintere Abschnitt (3) zwei Gewinde (30, 31) jeweils mit einer Steigung (A) aufweist, die mindestens das Doppelte ist von der Steigung (B) des Gewindes (10) des vorderen Abschnitts (1).

2. Knochenschraube gemäß Anspruch 1, **dadurch gekennzeichnet, daß** der mittlere glatte Abschnitt (2) eine Länge aufweist, die ungefähr ein Drittel der gesamten Länge der Schraube ist.

3. Knochenschraube gemäß Anspruch 1 oder 2, **dadurch gekennzeichnet, daß** die hinteren Enden der vorderen und hinteren Abschnitte (1) selbstschneidend und selbstbohrend sind, das heißt, daß sie abgeschrägt sind und jede drei longitudinale Einschnitte (11, 33) aufweisen, die tief bis in die Sohlen (12, 13) dieser Abschnitte (1, 3) reichen.

4. Knochenschraube gemäß einem der Ansprüche 1 bis 3, **dadurch gekennzeichnet, daß** der hintere Abschnitt abgeschrägt ist mit einem Winkel, der gleich oder doppelt ist dem von dem vorderen Abschnitt.

5. Knochenschraube gemäß einem der Ansprüche 1 bis 4, **dadurch gekennzeichnet, daß** der vordere Abschnitt mit ungefähr 60° abgeschrägt ist und der hintere Abschnitt mit ungefähr 120° abgeschrägt ist.

6. Knochenschraube gemäß einem der Ansprüche 1 bis 3, **dadurch gekennzeichnet, daß** der vordere und der hintere Abschnitt jeder mit ungefähr 90° abgeschrägt sind.

7. Knochenschraube gemäß einem der Ansprüche 1 bis 2, **dadurch gekennzeichnet, daß** der hintere Abschnitt mit einem Winkel zwischen 20 und 50° abgeschliffen ist und keine Einschnitte aufweist.

8. Knochenschraube gemäß einem der Ansprüche 1 bis 6, **dadurch gekennzeichnet, daß** der Durchmesser der Sohle (12) des vorderen Abschnitts (1) gleich dem ganz außerhalb des hinteren Abschnitts (3) ist.
